# EUROPÄISCHE PATENTSCHRIFT

(11) **EP 3 324 198 B1**
(45) Veröffentlichungstag und Bekanntmachung des Hinweises auf die Patenterteilung: **25.11.2020**
(21) Anmeldenummer: 17196519.7
(22) Anmeldetag: 16.10.2017
(51) Int. Cl.: G01R 33/34, G01R 33/3415

(54) **HF-SPULENANORDNUNG UMFASSEND EINE FLEXIBLE LOKALSPULE UND EINE STARRE LOKALSPULE**
RF COIL ASSEMBLY COMPRISING A FLEXIBLE LOCAL COIL AND A RIGID LOCAL COIL
ENSEMBLE DE BOBINES RF COMPRENANT UNE BOBINE LOCALE FLEXIBLE ET UNE BOBINE LOCALE RIGIDE

(30) Priorität: 17.11.2016 DE 102016222635
(43) Veröffentlichungstag der Anmeldung: 23.05.2018
(73) Patentinhaber: Siemens Healthcare GmbH, 91052 Erlangen (DE)
(72) Erfinder: Xie, Jingyi, 91052 Erlangen (DE); Zink, Stephan, 91054 Erlangen (DE)

(56) Entgegenhaltungen:
- DE-A1- 10 011 522
- DE-A1-102004 055 332
- DE-A1-102005 053 280
- DE-A1-102011 007 065
- JP-A- H06 254 069
- US-A- 4 897 604
- US-B1- 6 980 002

## Beschreibung

In der Medizintechnik stellen bildgebende Verfahren wichtige Hilfsmittel dar. So zeichnet sich etwa die Bildgebung mittels Magnetresonanz (kurz MR), auch Magnetresonanztomographie (kurz MRT) genannt, durch hohe und variable Weichteilkontraste aus. Hierbei senden angeregte Kerne beim Relaxieren hochfrequente elektromagnetische Magnetresonanzsignale, die mittels elektrisch leitender Schleifen, sogenannter Spulen und/oder Antennen, empfangen werden können. Beim Empfangen wird durch das Magnetresonanzsignal eine Spannung in der Spule induziert. Diese Spulen werden möglichst nahe an einem Untersuchungsbereich, insbesondere einem Patienten oder einem Teil des Patienten, angeordnet. Insbesondere kann es vorteilhaft sein, wenn die Spulen den Untersuchungsbereich wenigstens teilweise umschließen. Deshalb werden diese Spulen auch als Lokalspulen (ein englischer Fachbegriff ist "local coils") bezeichnet.

Üblicherweise werden komplett starre Lokalspulen verwendet. Diese können jedoch nicht an die Form und das Volumen eines Untersuchungsbereichs angepasst werden. Dadurch kann nicht sichergestellt werden, dass die Lokalspulen möglichst nahe an dem Untersuchungsbereich angeordnet sind. Zwar können verschiedene Lokalspulen unterschiedlicher Größe vorgehalten werden, um für Untersuchungsbereiche mit unterschiedlicher Form oder unterschiedlichem Volumen eine möglichst passende Lokalspule auszuwählen. Dieses Vorgehen verursacht aber hohe Kosten, da mehrere Lokalspulen vorgehalten werden müssen. Außerdem ist dieses Vorgehen zeitlich ineffizient, besonders wenn mehrere Lokalspulen am Patienten getestet werden müssen, bis die geeignete Lokalspule gefunden wird. Weiterhin ist es bekannt, flexible Lokalspulen zu verwenden. Diese flexiblen Lokalspulen können entweder auf einen Untersuchungsbereich aufgelegt werden, oder um einen Untersuchungsbereich gewickelt werden. Ein Nachteil dieser bekannten flexiblen Lokalspulen ist, dass sie leicht vom vorgesehenen Untersuchungsbereich verrutschen. Weiterhin können im Falle eines Umwickelns eines Untersuchungsbereichs Überlagerungen von einzelnen Antennenelementen der Lokalspule vorkommen, es kann aber auch der Untersuchungsbereich nicht vollständig umschlossen sein, beides ist nachteilig für die Signalerfassung und damit die Bildqualität. Weiterhin stellt das Umwickeln eines Untersuchungsbereichs mit der flexiblen Lokalspule einen sehr aufwändigen Arbeitsschritt dar.

Aus den Druckschriften DE 100 11 522 A1, DE 10 2004 055332 A1 und US 6 980 002 B1 sind Spulenanordnungen umfassend eine flexible Lokalspule und eine starre Lokalspule bekannt, die miteinander verbunden werden können. Aus der Druckschrift DE 10 2005 053280 A1 ist darüber hinausgehend bekannt, die flexible Lokalspule unterschiedlich stark gekrümmt mit der starren Lokalspule zu verbinden. Aus der Druckschrift US 4 897 604 A ist bekannt, flexible Lokalspulen durch Brückenelemente zu vergrößern. Aus der Druckschrift JP H06 254069 A ist eine starre Lokalspule mit mehrfachen Aufnahmevorrichtungen für flexible Lokalspulen bekannt. Diese Spulenanordnungen sind aber nur in einem begrenzten Umfang an die Form oder das Volumen eines Untersuchungsbereiches anpassbar.

Es ist daher die Aufgabe der vorliegenden Erfindung, eine Spulenanordnung bereitzustellen, die schnell und kostengünstig an die Form oder das Volumen eines Untersuchungsbereiches anpassbar ist.

Die Aufgabe wird durch die Spulenanordnung nach dem unabhängigen Anspruch gelöst. Vorteilhafte Ausgestaltungen sind in den Unteransprüchen beschrieben.

Die Erfindung betrifft eine Spulenanordnung zum Empfangen von Hochfrequenzstrahlung, wobei die Spulenanordnung wenigstens eine flächig ausgebildete flexible Lokalspule sowie eine halbröhrenförmig ausgebildete starre Lokalspule umfasst. Die flexible Lokalspule umfasst hierbei flächig ausgebildete erste Antennenelemente zum Empfangen von Hochfrequenzstrahlung, sowie wenigstens zwei erste Verbindungselemente auf unterschiedlichen Seiten der flexiblen Lokalspule. Die starre Lokalspule umfasst hierbei zweite Antennenelemente zum Empfangen von Hochfrequenzstrahlung, sowie wenigstens zwei Aufnahmeelemente. Hierbei sind die ersten Verbindungselemente und die Aufnahmeelemente dazu ausgelegt, die flexible Lokalspule mit der starren Lokalspule zu einer röhrenförmigen Gesamtlokalspule zu verbinden. Die Spulenanordnung kann insbesondere dazu ausgelegt sein, in einem Magnetresonanztomographen (kurz MR-Gerät) eingesetzt zu werden. Hierbei sind unterschiedliche Seiten der flexiblen Lokalspule insbesondere gegenüberliegende Seiten der flexiblen Lokalspule.

Die Erfinder haben erkannt, dass die beschriebene Spulenanordnung durch die Flexibilität der flexiblen Lokalspule an die Form oder das Volumen anpassbar ist. Weiterhin erfolgt diese Anpassung schnell und kostengünstig, da die starre Lokalspule zusammen mit den Verbindungselementen eine effiziente Justierung der röhrenförmigen Gesamtlokalspule erlaubt. Weiterhin ist es möglich, dass in verschiedenen Varianten der Erfindung verschiedene flexible Lokalspulen mit derselben starren Lokalspule unterschiedliche Gesamtlokalspulen ausbilden. Da trotz der verschiedenen Gesamtlokalspulen immer die gleiche starre Lokalspule verwendet wird, ist diese Spulenanordnung kostengünstiger, als für unterschiedliche Formen und Volumina von Untersuchungsbereichen verschiedene komplette Spulen vorzuhalten.

Die Erfinder haben weiterhin erkannt, dass durch die Verbindung einer flexiblen Lokalspule mit einer starren Lokalspule schnell und stabil eine Gesamtlokalspule erstellt werden kann, die einen Untersuchungsbereich umgibt. Hierbei erzeugt die starre Lokalspule Steifigkeit und Rutschfestigkeit, insbesondere wenn der zu untersuchende Körperteil auf der starren Lokalspule gelagert wird. Durch diese Steifigkeit und Rutschfestigkeit kann die flexible Lokalspule einfach und schnell mit der starren Lokalspule verbunden werden.

Erfindungsgemäß umfasst die flexible Lokalspule weiterhin wenigstens zwei zweite Verbindungselemente. Hierbei sind die ersten Verbindungselemente und die Aufnahmeelemente dazu ausgelegt, die flexible Lokalspule in einer ersten Ausrichtung mit der starren Lokalspule zu einer ersten röhrenförmigen Gesamtlokalspule zu verwenden. Weiterhin sind die zweiten Verbindungselemente und die Aufnahmeelemente dazu ausgelegt, die flexible Lokalspule in einer zweiten Ausrichtung mit der starren Lokalspule zu einer zweiten röhrenförmigen Gesamtlokalspule zu verbinden. Hierbei unterscheidet sich die erste Ausrichtung der flexiblen Lokalspule von der zweiten Ausrichtung der flexiblen Lokalspule. Insbesondere kann relativ zur starren Lokalspule die flexible Lokalspule in der ersten Ausrichtung orthogonal zur flexiblen Lokalspule in der zweiten Ausrichtung sein. Die Erfinder haben erkannt, dass es durch die in der ersten Ausrichtung gebildete röhrenförmige Gesamtlokalspule und durch die in der zweiten Ausrichtung gebildete röhrenförmige zweite Gesamtlokalspule möglich ist, die Spulenanordnung an unterschiedliche Formen und/oder Volumina von Untersuchungsbereichen anzupassen, da sich die beiden Ausrichtungen erfindungsgemäss unterscheiden. Da hiermit die Spulenanordnung für mehrere Körperteile eines Patienten und/oder mehrere Patienten geeignet ist, kann mit dieser Spulenanordnung eine besonders große Flexibilität in der Anwendung erreicht werden.

Gemäß einem weiteren Aspekt der Erfindung umschließt die röhrenförmige Gesamtlokalspule ein erstes Untersuchungsvolumen, weiterhin umschließt die zweite röhrenförmige Gesamtlokalspule ein zweites Untersuchungsvolumen. Hierbei ist das zweite Untersuchungsvolumen größer oder gleich dem ersten Untersuchungsvolumen. Das zweite Untersuchungsvolumen ist insbesondere dann größer als das erste Untersuchungsvolumen, wenn die Volumenmaßzahl des zweiten Untersuchungsvolumens größer ist als die Volumenmaßzahl des ersten Untersuchungsvolumens. Durch diesen Aspekt der Erfindung ist es möglich, die Spulenanordnung besonders gut an unterschiedliche Volumina von Untersuchungsbereichen anzupassen, und somit für die Bildgebung von unterschiedlichen Körperteilen eines Patienten und/oder für unterschiedliche Patienten zu verwenden. Beispielsweise kann so die gleiche Spulenanordnung sowohl für ein Kind als auch für einen Erwachsenen verwendet werden. Weiterhin kann so die gleiche Spulenanordnung für unterschiedliche Teile des menschlichen Körpers verwendet werden, beispielsweise für den Ellenbogen und das Knie. Diese Aspekte führen dazu, dass diese Spulenanordnung kostengünstiger herzustellen ist als andere Spulenanordnungen mit dem gleichen Einsatzspektrum.

Gemäß einem weiteren Aspekt der Erfindung weist die flexible Lokalspule zwischen den ersten Verbindungselementen eine erste Anzahl von Antennenelementen zum Empfangen von Hochfrequenzstrahlung auf, und zwischen den zweiten Verbindungselementen eine zweite Anzahl von Antennenelementen zum Empfangen von Hochfrequenzstrahlung auf. Weiterhin ist nach diesem Aspekt der Erfindung die zweite Anzahl größer oder gleich der ersten Anzahl. Ein Antennenelement liegt insbesondere zwischen den ersten Verbindungselementen oder den zweiten Verbindungselementen, wenn es zumindest teilweise im von den ersten Verbindungselementen oder den zweiten Verbindungselementen aufgespannten Rechteck liegt. Die Erfinder haben erkannt, dass es durch diese Wahl der ersten und zweiten Anzahl möglich ist, für unterschiedliche Formen und/oder Volumina von Untersuchungsbereichen eine gute Qualität der Bildgebung zu erreichen, insbesondere mit wenigen Artefakten und Störungen. Insbesondere können Antennenelemente, die nicht zwischen den zweiten Verbindungselementen liegen, ausgebildet sein, abgeschaltet zu werden. Diese Antennenelemente nehmen in vielen Untersuchungssituationen nur Bilddaten mit schlechter Qualität auf.

Gemäß einem weiteren Aspekt der Erfindung kann die flexible Lokalspule auch entlang des Umlaufs der ersten röhrenförmigen Gesamtlokalspule zwischen jeweils zwei unterschiedlichen Seiten der flexiblen Lokalspule eine erste Anzahl von ersten Antennenelementen zum Empfangen von Hochfrequenzstrahlung aufweisen, und entlang des Umlaufs der zweiten röhrenförmigen Gesamtlokalspule zwischen jeweils zwei unterschiedlichen Seiten der flexiblen Lokalspule eine zweite Anzahl von ersten zum Empfangen von Hochfrequenzstrahlung aufweisen, wobei die zweite Anzahl größer als die erste Anzahl ist. Hierbei beinhaltet der Umlauf entlang einer röhrenförmigen Gesamtlokalspule insbesondere den Teil der flexiblen Lokalspule, der in der röhrenförmigen Gesamtlokalspule mit der starren Lokalspule eine vollständige Röhre ausbildet.

Gemäß einem weiteren Aspekt der Erfindung erstreckt sich die flexible Lokalspule flächig entlang einer ersten Achse und einer zweiten Achse, wobei die flexible Lokalspule in der ersten Ausrichtung entlang der ersten Achse gekrümmt ist, und wobei die flexible Lokalspule in der zweiten Ausrichtung entlang der zweiten Achse gekrümmt ist. Die Erfinder haben erkannt, dass durch Krümmen einer flächigen flexiblen Lokalspule eine halbröhrenförmige flexible Lokalspule entsteht, die besonders einfach und schnell mit einer halbröhrenförmigen starren Lokalspule verbindbar ist. Durch die Krümmung um zwei verschiedene Achse ist es weiterhin möglich, unterschiedliche Formen und/oder Volumina von Untersuchungsbereichen einzuschließen.

Nach einem weiteren Aspekt der Erfindung ist die erste Achse orthogonal zur zweiten Achse. Hierbei ergibt sich ein besonders großer Unterschied zwischen der Form und/oder der Größe des von der ersten röhrenförmigen Gesamtlokalspule umschlossenen ersten Untersuchungsvolumens und der Form und/oder der Größe des von der zweiten röhrenförmigen Gesamtlokalspule umschlossenen zweiten Untersuchungsvolumens. Dies impliziert, dass besonders unterschiedliche Formen und/oder Volumina von Untersuchungsbereichen kostengünstig mit nur einer Spulenanordnung untersucht werden können.

Gemäß einem weiteren Aspekt der Erfindung ist die Ausdehnung der flexiblen Lokalspule entlang der ersten Achse größer als die Ausdehnung der flexiblen Lokalspule entlang der zweiten Achse. Die Erfinder haben erkannt, dass mit einer derart ausgebildeten flexiblen Lokalspule insbesondere unterschiedliche Volumina von Untersuchungsbereichen untersucht werden können. Damit können kostengünstig mit nur einer Lokalspule verschiedene Teile eines Patienten oder Patienten mit unterschiedlicher Körpergröße und/oder unterschiedlichem Körpergewicht untersucht werden.

Gemäß einem weiteren Aspekt der Erfindung weist die flexible Lokalspule ein Flächenaspektverhältnis zwischen 1:1 und 1:2, insbesondere zwischen 9:10 und 1:2, insbesondere zwischen 4:5 und 1:2 auf. Die Erfinder haben erkannt, dass bei einem solchen Flächenaspektverhältnis der Unterschied zwischen der Ausdehnung entlang verschiedener Achsen groß genug ist, um unterschiedliche Größen und/oder Volumina von Untersuchungsbereichen bei unterschiedlichen Patienten und/oder unterschiedlichen Körperteilen zu erfassen. Gleichzeitig ist der Unterschied zwischen der Ausdehnung entlang verschiedener Achsen klein genug, dass keine weitere flexible Lokalspule benötigt wird, um einen Untersuchungsbereich geeignet abzubilden, wobei die Größe des Volumen dieses Untersuchungsbereichs zwischen der Größe des Untersuchungsvolumen der ersten röhrenförmigen Gesamtlokalspule und der Größe des Volumen der zweiten röhrenförmigen Gesamtspule liegt. Mit anderen Worten ist mit einem solchen Flächenaspektverhältnis der flexiblen Lokalspule keine weitere flexible Lokalspule mit einer Zwischengröße notwendig. Dies führt dazu, dass die Spulenanordnung besonders und effizient verwendet werden kann, insbesondere wenn ausprobiert werden muss, welche Größe einer Lokalspule für einen bestimmten Untersuchungsbereich geeignet ist.

Gemäß einem weiteren Aspekt der Erfindung ist jeweils mindestens ein erstes Verbindungselement an den zur ersten Achse parallelen Seiten der flexiblen Lokalspule angeordnet. Weiterhin ist jeweils mindestens ein zweites Verbindungselement an den zur zweiten Achse parallelen Seiten der flexiblen Lokalspule angeordnet. Durch die beschriebene Anordnung der ersten Verbindungselemente und der zweiten Verbindungselemente ist die flexible Lokalspule besonders schnell mit der starren Lokalspule verbindbar.

Gemäß einem weiteren Aspekt der Erfindung ist die flexible Lokalspule flächig kreuzförmig ausgebildet. Weiterhin sind ein erster Kreuzbalken der flexiblen Lokalspule parallel zur ersten Achse und ein zweiter Kreuzbalken der flexiblen Lokalspule parallel zur zweiten Achse. Hierbei weisen der erste Kreuzbalken und der zweite Kreuzbalken die gleiche Breite auf. Weiterhin sind die ersten Verbindungselemente als Enden des ersten Kreuzbalkens und die zweiten Verbindungselemente als Enden des zweiten Kreuzbalkens ausgebildet. Die Erfinder haben erkannt, dass es durch diese kreuzförmige Struktur gleichzeitig kostengünstig herstellbar ist, da die Verbindungselemente durch die Form der flexiblen Lokalspule entstehen und nicht während der Produktion als separate Teile mit der flexiblen Lokalspule verbunden werden müssen. Gleichzeitig ist diese kreuzförmige Struktur einfach und schnell anwendbar, da die Verbindungselemente als Teil der Struktur besonders robust sind.

Nach einem weiteren Aspekt der Erfindung sind die flexible Lokalspule sowie die starre Lokalspule dazu ausgebildet, eine lösbare Verbindung einzugehen. Insbesondere können die ersten Verbindungselemente und/oder die zweiten Verbindungselemente dazu ausgebildet sein, eine lösbare Verbindung mit den Aufnahmeelementen einzugehen. Die Erfinder haben erkannt, dass durch eine lösbare Verbindung eine kostengünstige Wiederverwendung von flexibler Lokalspule und starrer Lokalspule möglich ist. Weiterhin kann mit einer lösbaren Verbindung die Gesamtlokalspule besonders Untersuchungsbereiche besonders eng umschließen, die ein Entfernen einer eng umschließenden Gesamtlokalspule im Ganzen nicht ermöglichen, insbesondere das Knöchelgelenk, das Handgelenk oder die Halswirbelsäule.

Nach einem weiteren Aspekt der Erfindung sind die flexible Lokalspule sowie die starre Lokalspule dazu ausgebildet, eine Steckverbindung einzugehen. Insbesondere können die ersten Verbindungselemente und/oder die zweiten Verbindungselemente dazu ausgebildet sein, eine Steckverbindung mit den Aufnahmeelementen einzugehen. Durch eine Steckverbindung sind die flexible Lokalspule und die starre Lokalspule besonders effizient und schnell verbindbar.

Gemäß einem weiteren Aspekt der Erfindung ist die Spulenanordnung derart ausgebildet, dass die flexible Lokalspule weiterhin erste Sicherungselemente umfasst und/oder die starre Lokalspule weiterhin zweite Sicherungselemente umfasst. Hierbei sind die flexible Lokalspule sowie die starre Lokalspule dazu ausgebildet, mittels der ersten und/oder zweiten Sicherungselemente eine gesicherte Verbindung einzugehen. Die ersten Sicherungselemente können in die ersten Verbindungselemente und/oder in die zweiten Verbindungselemente integriert sein. Die zweiten Sicherungselemente können in die Aufnahmeelemente integriert sein. Durch eine gesicherte Verbindung kann ein unabsichtliches Lösen der Verbindung und/oder ein unabsichtliches Verschieben der flexiblen Lokalspule verhindert werden.

Gemäß einem weiteren Aspekt der Erfindung sind die flexible Lokalspule sowie die starre Lokalspule jeweils dazu ausgebildet, als eine gesicherte Verbindung eine Klickverbindung, eine Schnappverbindung, eine Spannverbindung, und/oder eine Dornschließverbindung einzugehen. Insbesondere sind die ersten Sicherungselemente und/oder die zweiten Sicherungselemente dazu ausgebildet, eine Klickverbindung, eine Schnappverbindung, eine Spannverbindung, und/oder eine Dornschließverbindung auszubilden. Die Erfinder haben erkannt, dass die genannten Verbindungsarten sehr kostengünstig herzustellen sind, und durch die Bedienperson besonders effizient und schnell zu bedienen sind.

Gemäß einem weiteren Aspekt der Erfindung sind die flexible Lokalspule sowie die starre Lokalspule dazu ausgebildet, zwischen einander wenigstens ein elektrisches und/oder magnetisches Signal auszutauschen, wenn sie eine röhrenförmige Gesamtlokalspule ausbilden. Die Erfinder haben erkannt, dass es durch diesen möglichen Signalaustausch nicht notwendig ist, sowohl die flexible Lokalspule als auch die starre Lokalspule mit einer Auswerteeinheit zu verbinden. Vielmehr genügt es, dass entweder die flexible Lokalspule oder die starre Lokalspule ausgebildet ist, mit einer Auswerteeinheit verbunden zu werden. Die Verbindung kann beispielsweise als Kabelverbindung oder als Drahtlosverbindung ausgebildet sein. Dadurch ist die Lokalspule, die nicht dazu ausgebildet ist, mit einer Auswerteeinheit verbunden zu werden, kostengünstiger herzustellen. Weiterhin ist es bei der Anwendung der Spulenanordnung schneller und effizienter, nur eine der beiden Lokalspulen zu verbinden.

Gemäß einem weiteren Aspekt der Erfindung sind wenigstens ein erstes Antennenelement der flexiblen Lokalspule und wenigstens ein zweites Antennenelement der starren Lokalspule durch geometrischen Überlapp induktiv entkoppelt, wenn die flexible Lokalspule und die starren Lokalspule eine röhrenförmige Gesamtlokalspule ausbilden. Die Erfinder haben erkannt, dass durch diese Art der Entkopplung das Übersprechen von Antennenelementen, insbesondere das Übersprechen zwischen dem ersten und dem zweiten Antennenelement, effizient und kostengünstig ohne weitere elektronischen Elemente unterdrückt werden kann.

Eine Lokalspule wird insbesondere als starr bezeichnet, falls sie ein Elastizitätsmodul von mehr als 0.1 kN / mm² = 0.1 GPa, insbesondere von mehr als 1 kN / mm² = 1 GPa, insbesondere von mehr als 10 kN / mm² = 10 GPa bezüglich einer beliebigen Achse aufweist. Eine Lokalspule wird insbesondere als flexibel bezeichnet, falls sie ein Elastizitätsmodul von weniger als 10 N / mm² = 10 MPa, insbesondere weniger als 1 N / mm² = 1 MPa, insbesondere weniger als 0.1 N / mm² = 0.1 MPa bezüglich einer beliebigen Achse aufweist.

Eine röhrenförmige Struktur kann auch entlang ihrer Verzugsrichtung gekrümmt sein. Eine Struktur ist insbesondere dann röhrenförmig, wenn mehr als die Hälfte, insbesondere mehr als 75%, insbesondere jeder der möglichen zweidimensionalen Schnitte bezüglich einer zur Vorzugsrichtung orthogonalen Schnittebene ein konvexes Loch, insbesondere ein kreisförmiges oder ein ellipsenförmiges Loch umfasst. Die Außenseite einer röhrenförmigen Struktur kann insbesondere auch als Prisma geformt sein. Eine röhrenförmige Struktur kann weiterhin Aussparungen umfassen oder gitterförmig ausgebildet sein. Eine röhrenförmige Struktur kann also insbesondere funktionsbedingte oder ästhetische Löcher oder Aussparungen aufweisen.

Eine halbröhrenförmige Struktur kann durch zwei Schnitte parallel zur Vorzugsrichtung aus einer röhrenförmigen Struktur hervorgehen. Eine Struktur ist insbesondere dann halbröhrenförmig, wenn mehr als die Hälfte, insbesondere mehr als 75%, insbesondere jeder der möglichen zweidimensionalen Schnitte einer zur Vorzugsrichtung orthogonalen Schnittebene eine kreisbogenförmige, ellipsenbogenförmige oder konvexbogenförmige Kante aufweist. Die Struktur ist insbesondere dann halbröhrenförmig, falls die kreisbogenförmige Kante eine Ausdehnung zwischen einem Viertelkreis und einem Dreiviertelkreis, zwischen einer Viertelellipse und einer Dreiviertelellipse oder zwischen einer Viertelkonvexstruktur und einer Dreiviertelkonvexstruktur aufweist.

Eine Lokalspule ist insbesondere flächig ausgebildet, wenn die Ausdehnung der Lokalspule bezüglich einer ersten Achse und die Ausdehnung der Lokalspule bezüglich einer zur ersten Achse senkrechten zweiten Achse deutlich größer, insbesondere mehr als doppelt so groß, insbesondere mehr als fünfmal so groß, insbesondere mehr als zehnmal so groß wie Ausdehnung bezüglich einer zur ersten Achse und zur zweiten Achse senkrechten dritten Achse ist.

Das Untersuchungsvolumen ist der dreidimensionale Raum, aus dem Magnetresonanzsignale mittels der röhrenförmigen Gesamtlokalspule empfangbar sind. Insbesondere umfasst das Untersuchungsvolumen das dreidimensionale Volumen, welches von der röhrenförmigen Gesamtlokalspule umschlossen wird, auch wenn es nur entlang eines Umlaufs um die Vorzugsrichtung der röhrenförmigen Gesamtlokalspule umschlossen ist, also insbesondere nicht an den beiden Enden der röhrenförmigen Gesamtlokalspule umschlossen ist.

Als Untersuchungsbereich wird die dreidimensionale Struktur bezeichnet, welche mittels der röhrenförmigen Gesamtlokalspule und einem MR-Gerät bildgebend untersucht werden soll. Bei einem Untersuchungsbereich kann es sich insbesondere um einen Teil eines menschlichen Körpers, oder um ein Phantom handeln. Der Untersuchungsbereich ist während der Untersuchung mittels einer röhrenförmigen Gesamtlokalspule Teil des Untersuchungsvolumens.

Ein Antennenelement umfasst einen elektrischen Leiter, der vorzugsweise ein Material mit einer hohen elektrischen Leitfähigkeit aufweist, wie z.B. Kupfer. Insbesondere kann ein Antennenelement auch eine einfach oder mehrfach gewickelte, geschlossene oder offene Leiterschleife umfassen, und insbesondere als eine Leiterspule ausgebildet sein. Ein Antennenelement ist ausgebildet, Hochfrequenzstrahlung in einem MR-Gerät zu empfangen. Weiterhin kann ein Antennenelement beispielsweise mittels induktiver Kopplung, aber auch mittels elektrischer Kopplung mit einem anderen Antennenelement gekoppelt sein.

Ein erstes Antennenelement und ein zweites Antennenelement sind insbesondere derart durch geometrischen Überlapp entkoppelt, dass der durch den Überlapp der Antennenelemente bedingte Überlappbereich so bemessen ist, dass der durch den Überlappbereich hindurchtretende magnetische Fluss gerade den in den anderen Bereichen von dem jeweils anderen Antennenelement erzeugten magnetischen Fluss aufhebt. Hochfrequenzstrahlung ist elektromagnetische Strahlung mit einer Frequenz von mehr als 1 MHz, insbesondere mehr als 10 MHz, insbesondere mehr als 100 MHz.

Man betrachte eine flexible Lokalspule, die sich flächig entlang einer ersten Achse und einer zweiten Achse erstreckt. Die erste Hauptachse der flexiblen Lokalspule ist die (nicht notwendigerweise mit der ersten oder der zweiten Achse identische) Achse, entlang derer die flexible Lokalspule die maximale Ausdehnung aufweist. Die zweite Hauptachse ist parallel zu der von der ersten Achse und der zweiten Achse aufgespannten Ebene und orthogonal zur ersten Hauptachse. Das Flächenaspektverhältnis der flexiblen Lokalspule ist das Verhältnis der Ausdehnung n entlang der zweiten Hauptachse zur Ausdehnung m entlang der ersten Hauptachse. Das Flächenaspektverhältnis wird dann oftmals als n:m oder als 1:(m/n) angegeben.

Im Folgenden wird die Erfindung anhand der in den Figuren dargestellten Ausführungsbeispiele näher beschrieben und erläutert.

Es zeigen:
- Fig. 1: eine perspektivische Ansicht einer starren Lokalspule
- Fig. 2: einen Schnitt durch eine starre Lokalspule
- Fig. 3: eine Aufsicht einer flexiblen Lokalspule
- Fig. 4: eine perspektivische Ansicht einer flexiblen Lokalspule
- Fig. 5: eine perspektivische Ansicht einer ersten röhrenförmigen Gesamtlokalspule
- Fig. 6: einen Schnitt durch eine erste röhrenförmige Gesamtlokalspule
- Fig. 7: eine perspektivische Ansicht einer zweiten röhrenförmigen Gesamtlokalspule
- Fig. 8: einen Schnitt durch eine zweite röhrenförmige Gesamtlokalspule
- Fig. 9: eine schematische Ansicht eines MR-Geräts mit einer Spulenanordnung

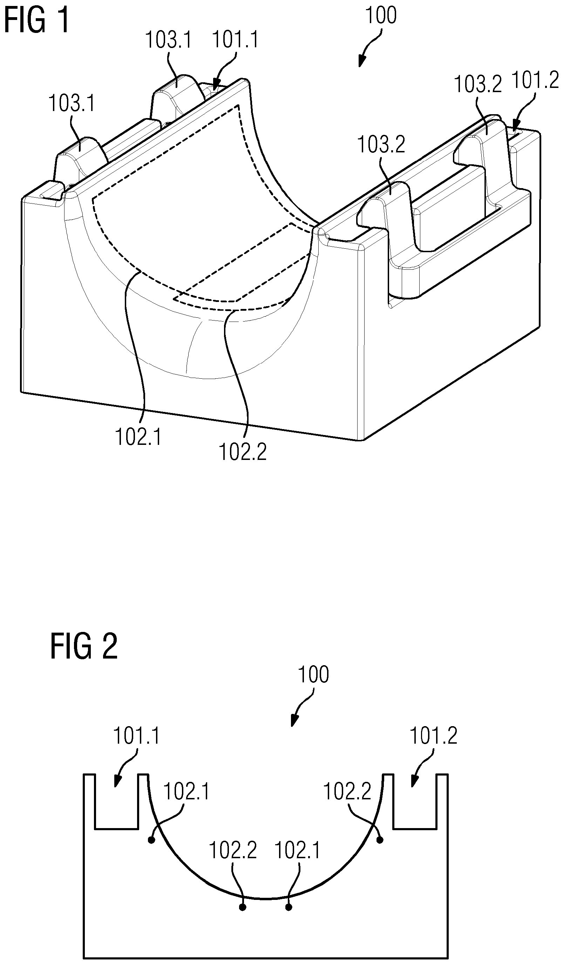

Fig. 1 zeigt ein Ausführungsbeispiel einer halbröhrenförmigen starren Lokalspule 100, Fig. 2 zeigt einen Schnitt durch die starre Lokalspule 100. Die Form der starren Lokalspule 100 entspricht auf ihrer Außenseite im Wesentlichen einem Quader und auf ihrer Innenseite einer Röhre. Die starre Lokalspule 100 weist Aufnahmeelemente 101.1, 101.2 auf. In dem gezeigten Ausführungsbeispiel sind die Aufnahmeelemente 101.1, 101.2 als Vertiefungen in den Körper der starren Lokalspule 100 ausgeführt. Die Aufnahmeelemente 101.1, 101.2 sind dazu ausgelegt, mit ersten Verbindungselementen 301.1, 301.2 oder zweiten Verbindungselementen 302.1, 302.2 einer flexiblen Lokalspule 300 verbunden zu werden. Im gezeigten Ausführungsbeispiel ist die Verbindung durch Einstecken der ersten Verbindungselemente 301.1, 301.2 oder der zweiten Verbindungselemente 302.1, 302.2 in die Aufnahmeelemente 101.1, 101.2 herstellbar.

Die starre Lokalspule 100 weist weiterhin zweite Antennenelemente 102.1, 102.2 auf. Die starre Lokalspule kann weitere zweite Antennenelemente aufweisen, die in Fig. 1 und Fig. 2 aus Gründen der Übersichtlichkeit nicht dargestellt sind. Die zweiten Antennenelemente 102.1, 102.2 sind in diesem Ausführungsbeispiel als einfach gewickelte Leiterschleifen ausgeführt. Sie können aber auch als mehrfach gewickelte Leiterschleifen ausgeführt sein. Die zweiten Antennenelemente 102.1, 102.2 sind dazu ausgebildet, Hochfrequenzstrahlung zu empfangen. Die zweiten Antennenelemente 102.1, 102.2 sind im gezeigten Ausführungsbeispiel durch geometrischen Überlapp entkoppelt. Im gezeigten Ausführungsbeispiel ist wenigstens ein zweites Antennenelement 102.1, 102.2 mit der Hochfrequenzantennensteuereinheit 911 verbunden, diese Verbindung und die Hochfrequenzantennensteuereinheit 911 sind in Fig. 1 und Fig. 2 nicht dargestellt. Alternativ kann wenigstens ein zweites Antennenelement 102.1, 102.2 indirekt über die flexible Lokalspule 300 mit der Hochfrequenzantennensteuereinheit 911 verbunden sein. Die starre Lokalspule 100 kann weiterhin elektronische Elemente zum Auslesen von Signalen der Antennenelemente 102.1, 102.2 oder zur Verarbeitung dieser Signale aufweisen, diese elektronischen Elemente sind nicht dargestellt.

Die starre Lokalspule 100 weist im gezeigten Ausführungsbeispiel weiterhin zweite Sicherungselemente 103.1, 103.2 auf. Die zweiten Sicherungselemente 103.1, 103.2 sind dazu ausgelegt, mit den ersten Sicherungselementen 304.1, 304.2, 305.1, 305.2 einer flexiblen Lokalspule 300 eine gesicherte Verbindung auszubilden. Die zweiten Sicherungselemente 103.1, 103.2 sind im gezeigten Ausführungsbeispiel dazu ausgelegt, mit den ersten Sicherungselementen 304.1, 304.2, 305.1, 305.2 eine Klickverbindung einzugehen. Die zweiten Sicherungselemente 103.1, 103.2 sind dabei derart als Laschen ausgebildet, und die ersten Sicherungselemente 304.1, 304.2, 305.1, 305.2 sind dabei derart als Vertiefungen ausgebildet, dass die Laschen in die Vertiefungen greifen und so die ersten Verbindungselemente 301.1, 301.2 der flexiblen Lokalspule 300 in den Aufnahmeelementen 101.1, 101.2 der starren Lokalspule fixieren. Zum Lösen der Verbindung können die Laschen mit einem Hebelmechanismus nach außen bewegt werden.

Neben der Klickverbindung sind noch andere Verbindungen möglich, beispielsweise eine Schnappverbindung, eine Spannverbindung, eine Dornschließverbindung oder eine Klettverbindung. Bei einer Schnappverbindung können die zweiten Sicherungselemente 103.1, 103.2 als Schnapphaken sowie die ersten Sicherungselemente 304.1, 304.2, 305.1, 305.2 als Widerstück zum Schnapphaken ausgebildet sein. Bei einer Spannverbindung können die ersten Sicherungselemente 304.1, 304.2, 305.1, 305.2 als Haken sowie die zweiten Sicherungselemente 103.1, 103.2 als Spannhebel ausgebildet sein. Bei einer Dornschließverbindung können die ersten Sicherungselemente 304.1, 304.2, 305.1, 305.2 als Band mit Löchern ausgebildet sein, und die zweiten Sicherungselemente 103.1, 103.2 als dazu passende Schließe mit Dorn. Natürlich können die ersten Sicherungselemente 304.1, 304.2, 305.1, 305.2 und die zweiten Sicherungselemente 103.1, 103.2 als jeweils anderer Teil dieser Verbindungen ausgebildet sein.

Die starre Lokalspule 100 kann insbesondere aus einem Kunststoff bestehen, in den zweite Antennenelemente 102.1, 102.2 eingebettet sind. Der Kunststoff kann insbesondere auch abwaschbar ausgebildet sein, insbesondere durch eine Oberflächenbeschichtung. Eine abwaschbare Ausführung ist besonders im Hinblick auf eine einfache und schnelle Reinigung vorteilhaft.

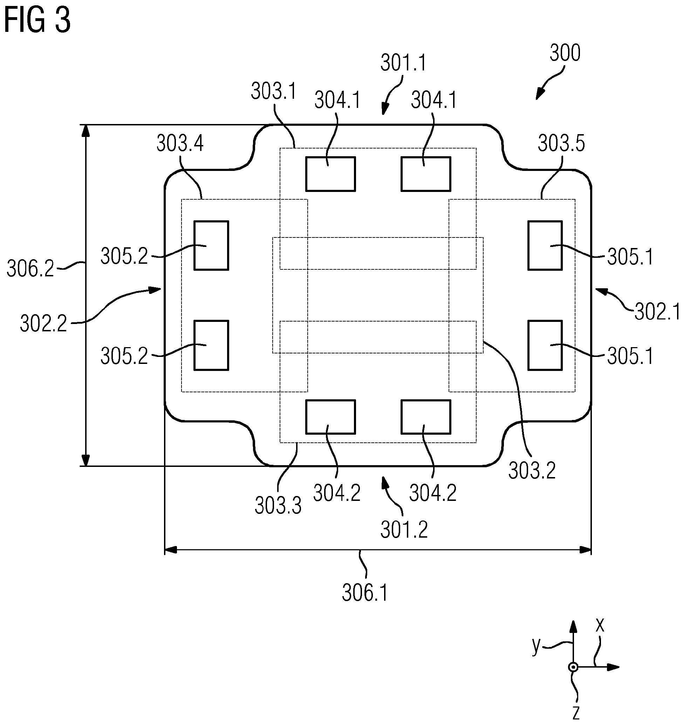

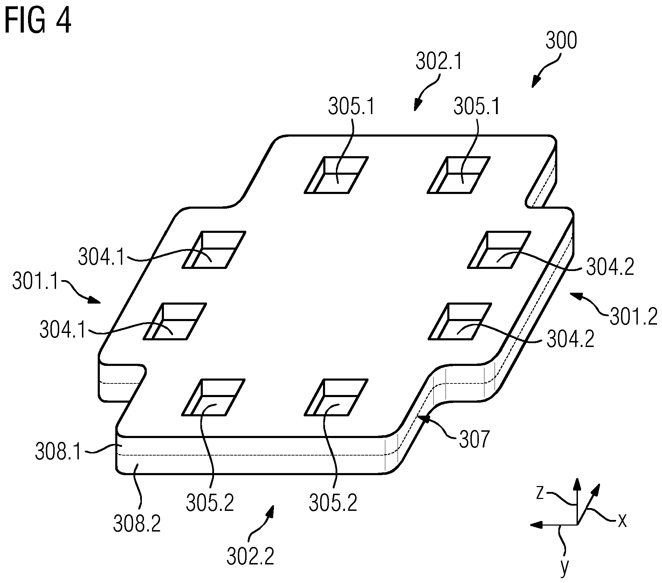

Fig. 3 zeigt eine Aufsicht eines Ausführungsbeispiels einer flächig ausgebildeten, flexiblen Lokalspule 300, Fig. 4 zeigt eine Schrägsicht desselben Ausführungsbeispiels einer flächig ausgebildeten, flexiblen Lokalspule 300. Die flexible Lokalspule ist hier flächig kreuzförmig ausgebildet. Es sind aber auch andere flächige Geometrien denkbar, weiterhin sind flächig kreuzförmige Geometrien denkbar, bei denen sich die Kreuzbalken nicht in ihrer Mitte überlagern. Die flexible Lokalspule 300 weist hier erste Verbindungselemente 301.1, 301.2 sowie zweite Verbindungselemente 302.1, 302.1 auf, die dazu ausgebildet sind, mit den Aufnahmeelementen 101.1, 101.2 der starren Lokalspule 100 durch Einstecken verbunden zu werden. Die ersten Verbindungselemente 301.1, 301.2 sowie die zweiten Verbindungselemente 302.1, 302.1 sind im dargestellten Ausführungsbeispiel als Enden jeweils eines Kreuzbalkens ausgeführt.

Die flexible Lokalspule 300 weist weiterhin erste Antennenelemente 303.1, 303.2, 303.3, 303.4, 303.5 auf. Die ersten Antennenelemente 303.1, 303.2, 303.3, 303.4, 303.5 sind in diesem Ausführungsbeispiel als einfach gewickelte Leiterschleifen ausgeführt. Sie können aber auch als mehrfach gewickelte Leiterschleifen ausgeführt sein. Die ersten Antennenelemente 303.1, 303.2, 303.3, 303.4, 303.5 können im gezeigten Ausführungsbeispiel durch geometrischen Überlapp entkoppelt sein. Beispielsweise kann das Antennenelement 303.1 von den Antennenelementen 303.2, 303.4 und 303.5 durch geometrischen Überlapp entkoppelt sein. Im gezeigten Ausführungsbeispiel ist wenigstens ein erstes Antennenelement 303.1, 303.2, 303.3, 303.4, 303.5 mit der Hochfrequenzantennensteuereinheit 911 verbunden, diese Verbindung und die Hochfrequenzantennensteuereinheit 911 sind in Fig. 3 und Fig. 4 nicht dargestellt. Alternativ kann wenigstens ein erstes Antennenelement 303.1, 303.2, 303.3, 303.4, 303.5 mittels der starren Lokalspule 100 mit der Hochfrequenzantennensteuereinheit 911 verbunden sein. Die flexible Lokalspule 300 kann weiterhin elektronische Elemente zum Auslesen von Signalen der ersten Antennenelemente 303.1, 303.2, 303.3, 303.4, 303.5 oder zur Verarbeitung dieser Signale aufweisen, diese elektronischen Elemente sind nicht dargestellt.

Die ersten Antennenelemente 303.1, 303.2, 303.3, 303.4, 303.5 und die zweiten Antennenelemente 102.1, 102.2 sind derart dazu ausgebildet, Hochfrequenzstrahlung zu empfangen, dass das elektromagnetische Wechselfeld der Hochfrequenzstrahlung gemäß den Maxwellgleichungen einen elektrischen Strom in wenigstens einem Teil eines Antennenelements 102.1, 102.2, 303.1, 303.2, 303.3, 303.4, 303.5 induziert. Dieser elektrische Strom kann dann gemessen werden.

Im gezeigten Ausführungsbeispiel weist die flexible Lokalspule 300 erste Sicherungselemente 304.1, 304.2 sowie zweite Sicherungselemente 305.1, 305.2 auf. Diese ersten Sicherungselemente sind dazu ausgebildet, eine lösbare, gesicherte Verbindung mit den ersten Sicherungselementen 103.1 und 103.2 einzugehen.

Im gezeigten Ausführungsbeispiel ist die flexible Lokalspule 300 entlang einer ersten Achse X und entlang einer zweiten Achse Y flächig ausgebildet. Hierbei bedeutet flächig ausgebildet, dass die Ausdehnung 306.1 der flexiblen Lokalspule 300 bezüglich der ersten Achse X sowie die Ausdehnung 306.2 der flexiblen Lokalspule 300 bezüglich der zweiten Achse Y jeweils mindestens doppelt so groß ist wie die Ausdehnung der flexiblen Lokalspule 300 bezüglich einer dritte, hier nicht dargestellte Achse Z, wobei die dritte Achse orthogonal zur ersten Achse x sowie orthogonal zur zweiten Achse Y ist. Im hier gezeigten Ausführungsbeispiel ist die Ausdehnung 306.1 der flexiblen Lokalspule 300 entlang der ersten Achse X größer als die Ausdehnung 306.2 entlang der zweiten Achse Y. Weiterhin ist das abgebildete Ausführungsbeispiel einer flexiblen Lokalspule 300 flächig kreuzförmig ausgebildet. Das Flächenaspektverhältnis der flexiblen Lokalspule berechnet sich hier aus dem Verhältnis der Ausdehnung 306.1 entlang der ersten Achse X zur Ausdehnung 306.2 entlang der zweiten Achse Y.

Das gezeigte Ausführungsbeispiel hat entlang der ersten Achse Y eine Ausdehnung 306.1 von 40 cm und entlang der zweiten Achse Y eine Ausdehnung 306.2 von 30 cm. Damit ergibt sich ein ungefähres Flächenaspektverhältnis von 3:4 oder 1:1,33. Es ist selbstverständlich auch eine Vielzahl anderer Flächenaspektverhältnisse realisierbar.

Im dargestellten Ausführungsbeispiel der Fig. 3 und Fig. 4 weist die flexible Lokalspule 300 bezüglich einer Krümmung um die erste Achse X und bezüglich einer Krümmung um die zweite Achse Y eine neutrale Ebene 307 auf. Als neutrale Ebene 307 wird diejenige Ebene der flexiblen Lokalspule 300 bezeichnet, deren Fläche sich beim Krümmen um die erste Achse X oder um die zweite Achse Y nicht ändert. Eine neutrale Ebene 307 bezeichnet also die zweidimensionale Verallgemeinerung einer neutralen Faser. Im gezeigten Ausführungsbeispiel sind die Antennenelemente 303.1, 303.2, 303.3, 303.4, 303.5 in der neutralen Ebene 307 oder nahe an der neutralen Ebene 307 lokalisiert. Nahe an der neutralen Ebene 307 heißt hier, dass der Abstand eines Antennenelements 303.1, 303.2, 303.3, 303.4, 303.5 von der neutralen Ebene 307 weniger als 20% der maximalen Dicke der flexiblen Lokalspule 300 beträgt. Die maximale Dicke der flexiblen Lokalspule 300 bezeichnet hierbei die maximal Ausdehnung der flexiblen Lokalspule bezüglich einer dritten Achse Z, die senkrecht auf der von der ersten Achse X und der zweiten Achse Y aufgespannten Fläche steht.

Im gezeigten Ausführungsbeispiel besteht die flexible Lokalspule 300 aus einer ersten Schicht 308.1 und einer zweiten Schicht 308.2 flexiblen Materials, welche die gleiche Dicke aufweisen. Bei dem flexiblen Material kann es sich insbesondere um Schaumstoff handeln. Die Antennenelemente 303.1, 303.2, 303.3, 303.4, 303.5 sind dann zwischen der ersten Schicht 308.1 und der zweiten Schicht 308.2 angeordnet. Eine derartige Anordnung ermöglicht eine besonders effektive Aufnahme etwaiger mechanische Belastungen durch die flexiblen Schichten 308.1, 308.2 statt durch die Antennenelemente 303.1, 303.2, 303.3, 303.4, 303.5. Weiterhin kann die flexible Lokalspule 300 noch andere Bestandteile umfassen, beispielsweise ein flexibles Schutzgehäuse zum Schutz der ersten und der zweiten Schicht 308.1, 308.2. Das flexible Schutzgehäuse kann beispielsweise aus einem Stoffgewebe bestehen.

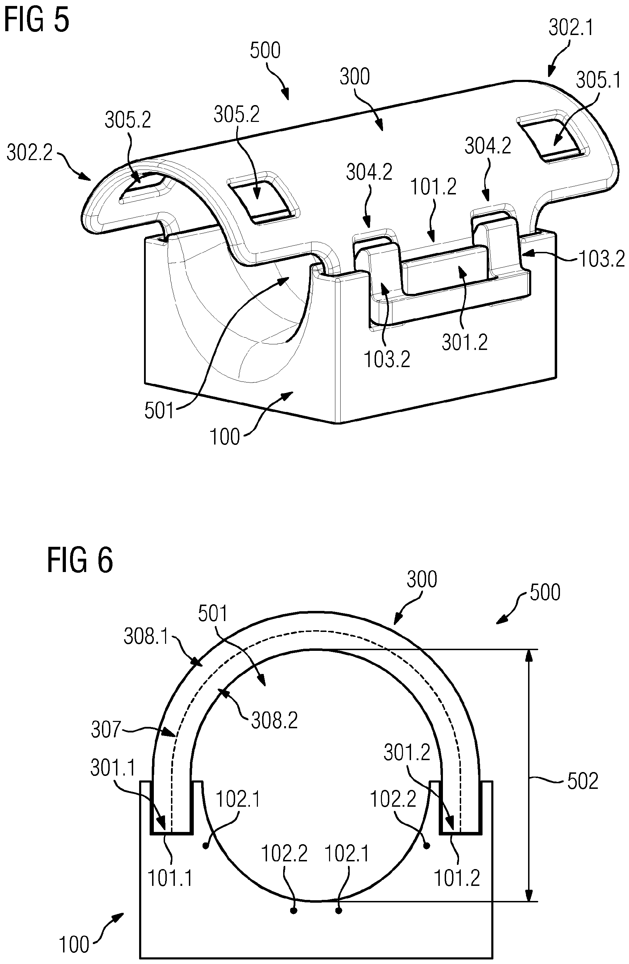

Fig. 5 zeigt ein Ausführungsbeispiel einer ersten röhrenförmigen Gesamtlokalspule 500, wobei hierbei die flexible Lokalspule 300 in einer ersten Ausrichtung mit der starren Lokalspule 100 verbunden ist. Fig. 6 zeigt einen Schnitt durch die erste röhrenförmige Gesamtlokalspule. Zur Konstruktion dieser Gesamtlokalspule 500 wird die flexible Lokalspule 300 bezüglich der ersten Achse X gebogen und in die starre Lokalspule 100 eingesteckt. Hierbei sind die ersten Verbindungselemente 301.1 und 301.2 in die Aufnahmeelemente 101.1, 101.2 eingesteckt, die Verbindung ist mittels der ersten Sicherungselemente 304.1, 304.2 und der zweiten Sicherungselemente 103.1, 103.2 gesichert. Die röhrenförmige Gesamtlokalspule umschließt hier ein Untersuchungsvolumen 501. In der gezeigten Konfiguration ist das (in Fig. 5 und Fig. 6 nicht dargestellte) erste Antennenelement 303.3 vom zweiten Antennenelement 102.2 der starren Lokalspule 100 durch geometrischen Überlapp entkoppelt. Weiterhin ist das (in Fig. 5 und Fig. 6 nicht dargestellte) erste Antennenelement 303.1 vom zweiten Antennenelement 102.1 durch geometrischen Überlapp entkoppelt. Alternativ sind auch andere galvanische, kapazitive und induktive Entkopplungstechniken vorstellbar. Im gezeigten Ausführungsbeispiel ist jeweils nur ein erstes Antennenelement 303.1, 303.3 von einem zweiten Antennenelement 102.1, 102.2 durch Überlapp entkoppelt. Alternativ können auch jeweils ein oder mehrere erste Antennenelemente von jeweils einem oder mehreren zweite Antennenelemente durch Überlapp entkoppelt sein. Im gezeigten Ausführungsbeispiel ist es möglich, einen Teil der (in Fig. 5 und Fig. 6 nicht dargestellten) ersten Antennenelemente 303.4, 303.5 nicht für die Datenaufnahme zu verwenden. In dieser Ausrichtung gibt es zu den ersten Antennenelemente 303.4, 303.5 keine zweiten Antennenelemente in der starren Lokalspule, die in einer Ebene senkrecht zur Vorzugsrichtung der röhrenförmigen Gesamtlokalspule zusammen mit den ersten Antennenelementen 303.4, 303.4 ein Untersuchungsvolumen vollständig umschließen. Daher würden die ersten Antennenelemente 303.4, 303.5 kein qualitativ zu den anderen ersten und zweiten Antennenelementen vergleichbares Signal messen können.

Im gezeigten Ausführungsbeispiel der Fig. 5 und Fig. 6 hat ein Schnitt des ersten Untersuchungsvolumens 501 senkrecht zur Vorzugsachse der röhrenförmigen Gesamtlokalspule 500 einen Umfang von 45 cm und damit einen mittleren Durchmesser 502 von circa 14,3 cm. Der Durchmesser bezüglich verschiedener Achsen durch den Schnitt kann aufgrund der Flexibilität der flexiblen Lokalspule variieren. Genauso sind natürlich andere Ausführungsbeispiele denkbar, bei denen sich andere Umfänge und Durchmesser ergeben.

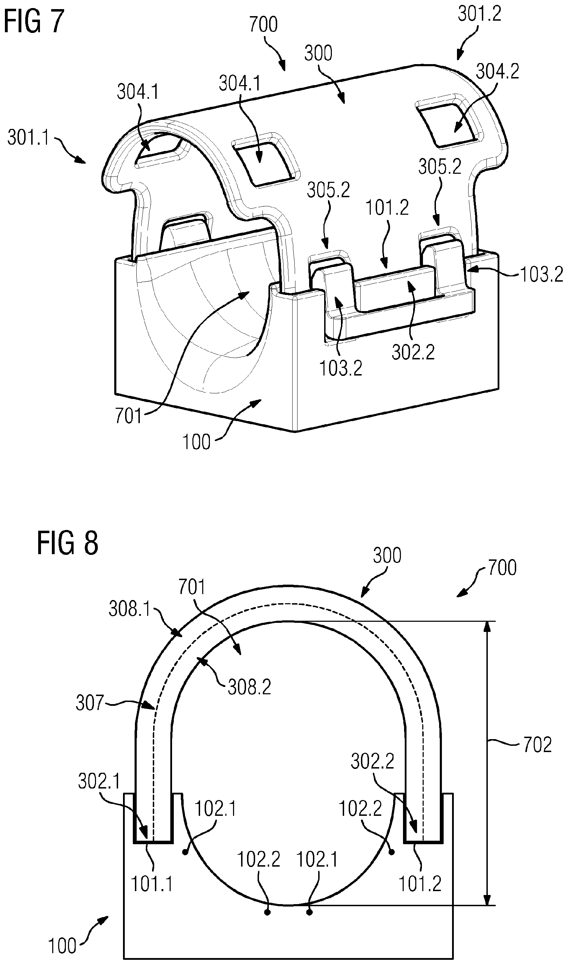

Fig. 7 zeigt ein Ausführungsbeispiel einer zweiten röhrenförmigen Gesamtlokalspule 700, wobei hierbei die flexible Lokalspule 300 in einer zweiten Ausrichtung mit der starren Lokalspule 100 verbunden ist, wobei die flexible Lokalspule 300 in der zweiten Ausrichtung im Vergleich zur flexiblen Lokalspule 300 in der ersten Ausrichtung um 90 Grad gedreht ist. Fig. 8 zeigt einen Schnitt durch die zweite röhrenförmige Gesamtlokalspule. Zur Konstruktion der zweiten röhrenförmigen Gesamtlokalspule 700 wird die flexible Lokalspule 300 bezüglich der zweiten Achse Y gebogen und in die starre Lokalspule 100 eingesteckt. Hierbei sind die zweiten Verbindungselemente 302.1 und 302.2 in die Aufnahmeelemente 101.1, 101.2 eingesteckt, die Verbindung ist mittels der ersten Sicherungselemente 305.1, 305.2 und der zweiten Sicherungselemente 103.1, 103.2 gesichert. Die röhrenförmige Gesamtlokalspule umschließt hier ein Untersuchungsvolumen 701. In der gezeigten Konfiguration ist das (in Fig. 7 und Fig. 8 nicht dargestellte) erste Antennenelement 303.5 vom zweiten Antennenelement 102.2 der starren Lokalspule 100 durch geometrischen Überlapp entkoppelt Weiterhin ist das (in Fig. 7 und Fig. 8 nicht dargestellte) erste Antennenelement 303.4 vom zweiten Antennenelement 102.1 durch geometrischen Überlapp entkoppelt. Alternativ sind auch andere kapazitive und induktive Entkopplungstechniken vorstellbar.. Im gezeigten Ausführungsbeispiel ist jeweils nur ein erstes Antennenelement 303.4, 303.5 von einem zweiten Antennenelement 102.1, 102.2 durch Überlapp entkoppelt. Alternativ können auch jeweils ein oder mehrere erste Antennenelemente von jeweils einem oder mehreren zweite Antennenelemente durch Überlapp entkoppelt sein. Im gezeigten Ausführungsbeispiel sind alle ersten Antennenelemente 303.1, 303.2, 303.3, 303.4, 303.5 der flexiblen Lokalspule 300 zur Datenaufnahmen verwendbar.

Im gezeigten Ausführungsbeispiel der Fig. 7 und Fig. 8 hat ein Schnitt des ersten Untersuchungsvolumens 701 senkrecht zur Vorzugsachse der röhrenförmigen Gesamtlokalspule 500 einen Umfang von 55 cm und damit einen mittleren Durchmesser 702 von circa 17.5 cm. Der Durchmesser bezüglich verschiedener Achsen durch den Schnitt kann aufgrund der Flexibilität der flexiblen Lokalspule variieren. Genauso sind natürlich andere Ausführungsbeispiele denkbar, bei denen sich andere Umfänge und Durchmesser ergeben.

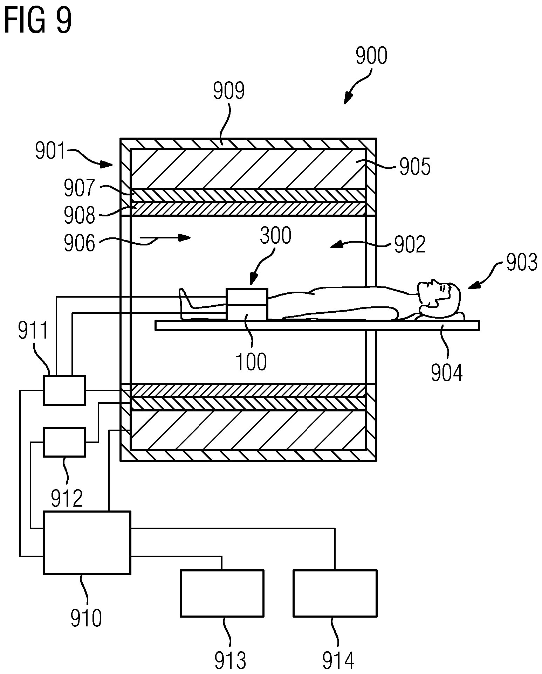

In Fig. 9 ist ein MR-Gerät 900 schematisch dargestellt. Das MR-Gerät 900 umfasst eine Magneteinheit 901, die einen Hauptmagneten 905 zu einem Erzeugen eines starken und insbesondere zeitlich konstanten Hauptmagnetfelds 906 aufweist. Zudem umfasst das MR-Gerät 900 einen Patientenaufnahmebereich 902 zu einer Aufnahme eines Patienten 903. Der Patientenaufnahmebereich 902 im vorliegenden Ausführungsbeispiel ist zylinderförmig ausgebildet und in einer Umfangsrichtung von der Magneteinheit 901 zylinderförmig umgeben. Grundsätzlich ist jedoch eine davon abweichende Ausbildung des Patientenaufnahmebereichs 902 jederzeit denkbar. Der Patient 903 kann mittels einer Patientenlagerungsvorrichtung 904 des MR-Geräts 900 in den Patientenaufnahmebereich 902 geschoben werden. Die Patientenlagerungsvorrichtung 904 weist hierzu einen innerhalb des Patientenaufnahmebereichs 902 bewegbar ausgestalteten Patiententisch auf. Die Magneteinheit 901 ist mittels einer Gehäuseverkleidung 909 des MR-Geräts 900 nach außen abgeschirmt.

Die Magneteinheit 901 weist weiterhin eine Gradientenspuleneinheit 907 zu einer Erzeugung von Magnetfeldgradienten auf, die für eine Ortskodierung während einer Bildgebung verwendet werden. Die Gradientenspuleneinheit 907 wird mittels einer Gradientensteuereinheit 912 des MR-Geräts 900 gesteuert. Die Magneteinheit 901 umfasst weiterhin eine Hochfrequenzantenneneinheit 908, welche im vorliegenden Ausführungsbeispiel als fest in das MR-Gerät 900 integrierte Körperspule ausgebildet ist. Die Hochfrequenzantenneneinheit 908 ist zu einer Anregung von Atomkernen, die sich in dem von dem Hauptmagneten 905 erzeugten Hauptmagnetfeld 906 einstellt, ausgelegt. Die Hochfrequenzantenneneinheit 908 wird von einer Hochfrequenzantennensteuereinheit 911 des MR-Geräts 900 gesteuert und strahlt hochfrequente Wechselfelder in einen Untersuchungsraum ein, der im Wesentlichen von einem Patientenaufnahmebereich 902 des MR-Geräts 900 gebildet ist. Die Hochfrequenzantenneneinheit 908 ist weiterhin zum Empfang von Magnetresonanzsignalen ausgebildet.

Zu einer Steuerung des Hauptmagneten 905, der Gradientensteuereinheit 907 und zur Steuerung der Hochfrequenzantennensteuereinheit 908 weist das MR-Gerät 900 eine Systemsteuereinheit 910 auf. Die Systemsteuereinheit 910 steuert zentral das MR-Gerät 900, wie beispielsweise das Durchführen einer vorbestimmten bildgebenden Gradientenechosequenz. Zudem umfasst die Systemsteuereinheit 910 eine nicht näher dargestellte Auswerteeinheit zu einer Auswertung von medizinischen Bilddaten, die während der Magnetresonanzuntersuchung erfasst werden. Des Weiteren umfasst das MR-Gerät 900 eine Benutzerschnittstelle, diese umfasst eine Anzeigeeinheit 913 und eine Eingabeeinheit 914, die jeweils mit der Systemsteuereinheit 910 verbunden sind. Steuerinformationen wie beispielsweise Bildgebungsparameter, sowie rekonstruierte Magnetresonanzbilder können auf der Anzeigeeinheit 913, beispielsweise auf zumindest einem Monitor, für ein medizinisches Bedienpersonal angezeigt werden. Mittels der Eingabeeinheit 914 können Informationen und/oder Parameter während eines Messvorgangs von dem medizinischen Bedienpersonal eingegeben werde.

In einem MR-Gerät 900 werden mittels einer Hochfrequenzantenneneinheit 908 Hochfrequenz-Pulse ausgesendet, was dazu führt, dass die Kernspins bestimmter, durch diese Hochfrequenz-Pulse resonant angeregter Atome um einen definierten Flipwinkel gegenüber den Magnetfeldlinien des Hauptmagnetfelds 906 verkippt werden. Bei der Relaxation der Kernspins wird Hochfrequenzstrahlung abgestrahlt, die mittels geeigneter Antennenelemente 102.1, 102.2, 303.1, 303.2, 303.3, 303.4, 303.5 einer Lokalspule 100, 300 empfangen und dann weiterverarbeitet werden.

Ferner ist auf der Patientenlagerungsvorrichtung 904, insbesondere auf dem Patiententisch, eine Spulenanordnung 100, 300 bestehend aus einer starren Lokalspule 100 und einer flexiblen Lokalspule 300 angeordnet, wobei die flexible Lokalspule 300 mit der starren Lokalspule 100 verbunden ist. Mit Hilfe der Spulenanordnung 100, 300 können insbesondere Hochfrequenzsignale empfangen und an die Hochfrequenzantennensteuereinheit 911 übertragen werden. Im gezeigten Ausführungsbeispiel sind sowohl die starre Lokalspule 100 als auch die flexible Lokalspule 300 direkt mit der Hochfrequenzantennensteuereinheit 911 verbunden. Diese Verbindung kann mittels eines Kabels ausgeführt sein, es ist aber auch denkbar, eine drahtlose Verbindung zu verwenden. Weiterhin ist es möglich, dass die starre Lokalspule 100 und die flexible Lokalspule 300 über Kontakte oder drahtlos Signale austauschen können, in diesem Fall muss nur eine der beiden Lokalspulen 100, 300 direkt mit der Hochfrequenzantennensteuereinheit 911 verbunden werden. Die Spulenanordnung 100, 300 ist in diesem Fall zur Untersuchung des Knies des Patienten 903 ausgebildet, sie kann aber auch für andere Teile des Patienten 903 wie beispielsweise den Ellenbogen oder den Kopf verwendet werden.

Eine übliche Untersuchung eines Köperteils mittels einer Spulenanordnung bestehend aus einer starren Lokalspule 100 und einer flexiblen Lokalspule 300 erfolgt mittels der folgenden Schritte: Zunächst wird die starre Lokalspule 100 auf der Patientenlagerungsvorrichtung 904 platziert. Weiterhin wird der Patient 903 derart auf der Patientenlagerungsvorrichtung 904 gelagert, dass der zu untersuchende Körperteil in der Öffnung der starren Lokalspule 100 liegt. Weiterhin wird die flexible Lokalspule 300 entlang der ersten Achse X oder entlang der zweiten Achse Y gekrümmt und mit der starren Lokalspule 100 verbunden. Anschließend erfolgt die Aufnahme des Bilddatensatzes mittels des MR-Geräts 900. Nach der Aufnahme des Bilddatensatzes kann die flexible Lokalspule 300 von der starren Lokalspule 100 entfernt werden.

## Patentansprüche

1. Spulenanordnung zum Empfangen von Hochfrequenzstrahlung,
- umfassend wenigstens eine flächig ausgebildete flexible Lokalspule (300) sowie eine halbröhrenförmig ausgebildete starre Lokalspule (100),
- wobei die flexible Lokalspule (300) flächig ausgebildete, erste Antennenelemente (303.1, 303.2, 303.3, 303.4, 303.5) zum Empfangen von Hochfrequenzstrahlung, sowie wenigstens zwei erste Verbindungselemente (301.1, 301.2) auf unterschiedlichen Seiten der flexiblen Lokalspule (300) umfasst,
- wobei die starre Lokalspule (100) zweite Antennenelemente (102.1, 102.2) zum Empfangen von Hochfrequenzstrahlung, sowie wenigstens zwei Aufnahmeelemente (101.1, 101.2) umfasst, wobei die ersten Verbindungselemente (301.1, 301.2) und die Aufnahmeelemente (101.1, 101.2) dazu ausgelegt sind, die flexible Lokalspule (300) in einer ersten Ausrichtung mit der starren Lokalspule (100) zu einer ersten röhrenförmigen Gesamtlokalspule (500) zu verbinden,
**dadurch gekennzeichnet, dass**
- die flexible Lokalspule (300) weiterhin wenigstens zwei zweite Verbindungselemente (302.1, 302.2) umfasst,
- wobei die zweiten Verbindungselemente (302.1, 302.2) und die Aufnahmeelemente (101.1, 101.2) dazu ausgelegt sind, die flexible Lokalspule (300) in einer zweiten Ausrichtung mit der starren Lokalspule (100) zu einer zweiten röhrenförmigen Gesamtlokalspule (700) zu verbinden,
wobei sich die erste Ausrichtung der flexiblen Lokalspule (300) von der zweiten Ausrichtung der flexiblen Lokalspule (300) unterscheidet.

2. Spulenanordnung nach Anspruch 1,
- wobei die erste röhrenförmige Gesamtlokalspule (500) ein erstes Untersuchungsvolumen (501) umschließt,
- wobei die zweite röhrenförmige Gesamtlokalspule (700) ein zweites Untersuchungsvolumen (701) umschließt,
- und wobei das zweite Untersuchungsvolumen (701) größer als das erste Untersuchungsvolumen (501) ist.

3. Spulenanordnung nach Anspruch 1 oder 2,
- wobei die flexible Lokalspule (300) zwischen den ersten Verbindungselementen (301.1, 301.2) eine erste Anzahl von ersten Antennenelementen (303.1, 303.2, 303.3) zum Empfangen von Hochfrequenzstrahlung aufweist,
- wobei die flexible Lokalspule (300) zwischen den zweiten Verbindungselementen (302.1, 302.2) eine zweite Anzahl von Antennenelementen (303.1, 303.2, 303.3, 303.4, 303.5) zum Empfangen von Hochfrequenzstrahlung aufweist,
- und wobei die zweite Anzahl größer oder gleich der ersten Anzahl ist.

4. Spulenanordnung nach einem der vorherigen Ansprüche,
- wobei sich die flexible Lokalspule (300) flächig entlang einer ersten Achse (X) und einer zweiten Achse (Y) erstreckt,
- wobei die flexible Lokalspule (300) in der ersten Ausrichtung entlang der ersten Achse (X) gekrümmt ist,
- wobei die flexible Lokalspule (300) in der zweiten Ausrichtung entlang der zweiten Achse (Y) gekrümmt ist.

5. Spulenanordnung nach Anspruch 4, wobei die erste Achse (X) orthogonal zur zweiten Achse (Y) ist.

6. Spulenanordnung nach einem der Ansprüche 4 oder 5, wobei die Ausdehnung (306.1) der flexiblen Lokalspule (300) entlang der ersten Achse größer ist als die Ausdehnung (306.2) der flexiblen Lokalspule (300) entlang der zweiten Achse (Y).

7. Spulenanordnung nach Anspruch 6, wobei die flexible Lokalspule (300) ein Flächenaspektverhältnis zwischen 1:1 und 1:2, insbesondere zwischen 9:10 und 1:2, insbesondere zwischen 4:5 und 1:2 aufweist.

8. Spulenanordnung nach einem der Ansprüche 4 bis 7,
- wobei jeweils mindestens ein erstes Verbindungselement (301.1, 301.2) an den zur ersten Achse (X) parallelen Seiten der flexiblen Lokalspule (300) angeordnet ist,
- und wobei jeweils mindestens ein zweites Verbindungselement (302.1, 302.2) an den zur zweiten Achse (Y) parallelen Seiten der flexiblen Lokalspule (300) angeordnet ist.

9. Spulenanordnung nach einem der Ansprüche 4 bis 8,
- wobei die flexible Lokalspule (300) flächig kreuzförmig ausgebildet ist,
- wobei ein erster Kreuzbalken der flexiblen Lokalspule (300) parallel zur ersten Achse (X) ist und ein zweiter Kreuzbalken der flexiblen Lokalspule (300) parallel zur zweiten Achse (Y) ist,
- wobei der erste Kreuzbalken und der zweite Kreuzbalken die gleiche Breite aufweisen,
- wobei die ersten Verbindungselemente (301.1, 301.2) als Enden des ersten Kreuzbalkens ausgebildet sind,
- und wobei die zweiten Verbindungselemente (302.1, 302.2) als Enden des zweiten Kreuzbalkens ausgebildet sind.

10. Spulenanordnung nach einem der vorherigen Ansprüche, wobei die flexible Lokalspule (300) sowie die starre Lokalspule (100) dazu ausgebildet sind, eine lösbare Verbindung einzugehen.

11. Spulenanordnung nach Anspruch 10, wobei die flexible Lokalspule (300) sowie die starre Lokalspule (100) dazu ausgebildet sind, eine Steckverbindung einzugehen.

12. Spulenanordnung nach Anspruch 11,
- wobei die flexible Lokalspule (300) weiterhin erste Sicherungselemente (304.1, 304.2, 305.1, 305.2) umfasst und/oder die starre Lokalspule (100) zweite Sicherungselemente (103.1, 103.2) umfasst,
- und wobei die flexible Lokalspule (300) sowie die starre Lokalspule (100) dazu ausgebildet sind, mittels der ersten Sicherungselemente (304.1, 304.2, 305.1, 305.2) und/oder der zweiten Sicherungselemente (103.1, 103.2) eine gesicherte Verbindung einzugehen.

13. Spulenanordnung nach Anspruch 12, wobei die flexible Lokalspule (300) sowie die starre Lokalspule (100) jeweils ausgebildet sind wenigstens eine der folgenden gesicherten Verbindungen einzugehen:
- Klickverbindung
- Schnappverbindung
- Spannverbindung
- Dornschließverbindung
- Klettverbindung

14. Spulenanordnung nach einem der vorherigen Ansprüche, wobei wenigstens ein erstes Antennenelement (303.1, 303.2, 303.3, 303.4, 303.5) der flexiblen Lokalspule (300) und wenigstens ein zweites Antennenelement (102.1, 102.2) der starren Lokalspule (100) durch geometrischen Überlapp induktiv entkoppelt sind, wenn die flexible Lokalspule (300) und die starre Lokalspule (100) eine röhrenförmige Gesamtlokalspule (500, 700) ausbilden.

## Claims

1. Coil arrangement for receiving radio-frequency radiation,
- comprising at least one flexible local coil (300) in a planar embodiment as well as a rigid local coil (100) in a semi-tubular embodiment,
- wherein the flexible local coil (300) comprises first antenna elements (303.1, 303.2, 303.3, 303.4, 303.5) in a planar embodiment for receiving radio-frequency radiation, as well as at least two first connection elements (301.1, 301.2) on different sides of the flexible local coil (300),
- wherein the rigid local coil (100) comprises second antenna elements (102.1, 102.2) for receiving radio-frequency radiation, as well as at least two receiving elements (101.1, 101.2),
wherein the first connection elements (301.1, 301.2) and the receiving elements (101.1, 101.2) are designed to connect the flexible local coil (300) in a first alignment to the rigid local coil (100) to form a first tubular overall local coil (500),
**characterised in that**
- the flexible local coil (300) also comprises at least two second connection elements (302.1, 302.2),
- wherein the second connection elements (302.1, 302.2) and the receiving elements (101.1, 101.2) are designed to connect the flexible local coil (300) in a second alignment to the rigid local coil (100) to form a second tubular overall local coil (700),
wherein the first alignment of the flexible local coil (300) differs from the second alignment of the flexible local coil (300) .

2. Coil arrangement according to claim 1,
- wherein the first tubular overall local coil (500) surrounds a first examination volume (501),
- wherein the second tubular overall local coil (700) surrounds a second examination volume (701),
- and wherein the second examination volume (701) is larger than the first examination volume (501).

3. Coil arrangement according to claim 1 or 2,
- wherein the flexible local coil (300), between the first connection elements (301.1, 301.2), has a first number of first antenna elements (303.1, 303.2, 303.3) for receiving radio-frequency radiation,
- wherein the flexible local coil (300), between the second connection elements (302.1, 302.2), has a second number of antenna elements (303.1, 303.2, 303.3, 303.4, 303.5) for receiving radio-frequency radiation,
- and wherein the second number is greater than or equal to the first number.

4. Coil arrangement according to one of the preceding claims,
- wherein the flexible local coil (300) extends in a planar manner along a first axis (X) and a second axis (Y),
- wherein the flexible local coil (300), in the first alignment, is curved along the first axis (X),
- wherein the flexible local coil (300), in the second alignment, is curved along the second axis (Y).

5. Coil arrangement according to claim 4, wherein the first axis (X) is orthogonal to the second axis (Y).

6. Coil arrangement according to one of claims 4 or 5, wherein the extent (306.1) of the flexible local coil (300) along the first axis is greater than the extent (306.2) of the flexible local coil (300) along the second axis (Y).

7. Coil arrangement according to claim 6, wherein the flexible local coil (300) has a surface aspect ratio of between 1:1 and 1:2, in particular between 9:10 and 1:2, in particular between 4:5 and 1:2.

8. Coil arrangement according to one of claims 4 to 7,
- wherein at least one first connection element (301.1, 301.2) is arranged in each case on the sides of the flexible local coil (300) parallel to the first axis (X),
- and wherein at least one second connection element (302.1, 302.2) in each case is arranged on the sides of the flexible local coil (300) parallel to the second axis (Y).

9. Coil arrangement according to one of claims 4 to 8,
- wherein the flexible local coil (300) is in a planar cross-shaped embodiment,
- wherein a first cross bar of the flexible local coil (300) is parallel to the first axis (X) and a second cross bar of the flexible local coil (300) is parallel to the second axis (Y),
- wherein the first cross bar and the second cross bar have the same width,
- wherein the first connection elements (301.1, 301.2) are embodied as ends of the first cross bar,
- and wherein the second connection elements (302.1, 302.2) are embodied as ends of the second cross bar.

10. Coil arrangement according to one of the preceding claims, wherein the flexible local coil (300), as well as the rigid local coil (100), are embodied to make a detachable connection.

11. Coil arrangement according to claim 10, wherein the flexible local coil (300), as well as the rigid local coil (100), are embodied to make a plug connection.

12. Coil arrangement according to claim 11,
- wherein the flexible local coil (300) further comprises first securing elements (304.1, 304.2, 305.1, 305.2) and/or the rigid local coil (100) comprises second securing elements (103.1, 103.2),
- and wherein the flexible local coil (300), as well as the rigid local coil (100), are embodied, by means of the first securing elements (304.1, 304.2, 305.1, 305.2) and/or the second securing elements (103.1, 103.2), to make a secured connection.

13. Coil arrangement according to claim 12, wherein the flexible local coil (300), as well as the rigid local coil (100), are each embodied to make at least one of the following secured connections:
- Click connection
- Snap-on connection
- Clamping connection
- Pin buckle connection
- Velcro connection

14. Coil arrangement according to one of the preceding claims, wherein at least one first antenna element (303.1, 303.2, 303.3, 303.4, 303.5) of the flexible local coil (300) and at least one second antenna element (102.1, 102.2) of the rigid local coil (100) are inductively decoupled by geometrical overlap when the flexible local coil (300) and the rigid local coil (100) form a tubular overall local coil (500, 700).

## Revendications

1. Agencement de bobines pour la réception de rayonnement de haute fréquence,
- comprenant au moins une bobine (300) locale souple, constituée en nappe, ainsi qu'une bobine (100) locale rigide, constituée en forme de demi-tube,
- dans lequel la bobine (300) locale souple comprend des premiers éléments (303.1, 303.2, 303.3, 303.4, 303.5) d'antenne, constitués en nappe, pour la réception du rayonnement de haute fréquence, ainsi qu'au moins deux premiers éléments (301.1, 301.2) d'assemblage sur des côtés différents de la bobine (300) locale souple,
- dans lequel la bobine (100) locale rigide comprend deux éléments (102.1, 102.2) d'antenne pour la réception du rayonnement de haute fréquence, ainsi qu'au moins deux éléments (101.1, 101.2) de réception, les premiers éléments (301.1, 301.2) d'assemblage et les éléments (101.1, 101.2) de réception étant conçus pour assembler la bobine (300) locale souple dans une première orientation à la bobine (100) locale rigide en une première bobine (500) locale d'ensemble en forme de tube,
**caractérisé en ce que**
- la bobine (300) locale souple comprend, en outre, au moins deux deuxièmes éléments (302.1, 302.2) d'assemblage,
- dans lequel les deuxièmes éléments (302.1, 302.2) d'assemblage et les éléments (101.1, 101.2) de réception sont conçus pour assembler la bobine (300) locale souple dans une deuxième orientation à la bobine (100) locale rigide en une deuxième bobine (700) locale d'ensemble en forme de tube,
dans lequel la première orientation de la bobine (300) locale souple est différente de la deuxième orientation de la bobine (300) locale souple.

2. Agencement de bobines suivant la revendication 1,
- dans lequel la première bobine (500) locale d'ensemble en forme de tube renferme un premier volume (501) à examiner,
- dans lequel la deuxième bobine (700) locale d'ensemble en forme de tube renferme un deuxième volume (701) à examiner,
- et dans lequel le deuxième volume (701) à examiner est plus grand que le premier volume (501) à examiner.

3. Agencement de bobines suivant la revendication 1 ou 2,
- dans lequel la bobine (300) locale souple a, entre les premiers éléments (301.1, 301.2) d'assemblage, un premier nombre de premiers éléments (303.1, 303.2, 303.3) d'antenne pour la réception du rayonnement de haute fréquence,
- dans lequel la bobine (300) locale souple a, entre les deuxièmes éléments (302.1, 302.2) d'assemblage, un deuxième nombre d'éléments (303.1, 303.2, 303.3, 303.4, 303.5) d'antenne pour la réception du rayonnement de haute fréquence,
- et dans lequel le deuxième nombre est supérieur ou égal au premier nombre.

4. Agencement de bobines suivant l'une des revendications précédentes,
- dans lequel la bobine (300) locale souple s'étend en nappe suivant un premier axe (X) et un deuxième axe (Y),
- dans lequel la bobine (300) locale souple est incurvée dans la première orientation suivant le premier axe (X),
- dans lequel la bobine (300) locale souple est incurvée dans la deuxième orientation suivant le deuxième axe (Y).

5. Agencement de bobines suivant la revendication 4, dans lequel le premier axe (X) est orthogonal au deuxième axe (Y).

6. Agencement de bobines suivant l'une des revendications 4 ou 5, dans lequel l'étendue (306.1) de la bobine (300) locale souple suivant le premier axe est plus grande que l'étendue (306.2) de la bobine (300) locale souple suivant le deuxième axe (Y).

7. Agencement de bobines suivant la revendication 6, dans lequel la bobine (300) locale souple a un rapport d'aspect en surface compris entre 1:1 et 1:2, notamment compris entre 9:10 et 1:2, notamment 4:5 et 1:2.

8. Agencement de bobines suivant l'une des revendications 4 à 7,
- dans lequel, respectivement, au moins un premier élément (301.1, 301.2) d'assemblage est disposé sur les côtés parallèles au premier axe (X) de la bobine (300) locale souple,
- et dans lequel, respectivement, au moins un deuxième élément (302.1, 302.2) d'assemblage est disposé sur les côtés parallèles au deuxième axe (Y) de la bobine (300) locale souple.

9. Agencement de bobines suivant l'une des revendications 4 à 8,
- dans lequel la bobine (300) locale souple est constituée en forme de croix en nappe,
- dans lequel une première traverse de la bobine (300) locale souple est parallèle au premier axe (X) et une deuxième traverse de la bobine (300) locale souple est parallèle au deuxième axe (Y),
- dans lequel la première traverse et la deuxième traverse ont la même largeur,
- dans lequel les premiers éléments (301.1, 301.2) d'assemblage sont constitués sous la forme d'extrémités de la première traverse,
- et dans lequel les deuxièmes éléments (302.1, 302.2) d'assemblage sont constitués sous la forme d'extrémités de la deuxième traverse.

10. Agencement de bobines suivant l'une des revendications précédentes, dans lequel la bobine (300) locale souple, ainsi que la bobine (100) locale rigide, sont constitués de manière à entrer en un assemblage amovible.

11. Agencement de bobines suivant la revendication 10, dans lequel la bobine (300) locale souple, ainsi que la bobine (100) locale rigide, sont constituées de manière à entrer en un assemblage par enfichage.

12. Agencement de bobines suivant la revendication 11,
- dans lequel la bobine (300) locale souple comprend, en outre, des premiers éléments (304.1, 304.2, 305.1, 305.2) de blocage et/ou la bobine (100) locale rigide comprend des deuxièmes éléments (103.1, 103.2) de blocage,
- et dans lequel la bobine (300) locale souple, ainsi que la bobine (100) locale rigide, sont constituées pour entrer, au moyen des premiers éléments (304.1, 304.2, 305.1, 305.2) de blocage et/ou des deuxièmes éléments (103.1, 103.2) de blocage, en un assemblage bloqué.

13. Agencement de bobines suivant la revendication 12, dans lequel la bobine (300) locale souple, ainsi que la bobine (100) locale rigide, sont constituées chacune pour entrer au moins en l'un des assemblages bloqués suivants :
- assemblage à déclic
- assemblage à encliquetage
- assemblage à serrage
- assemblage à fermeture à broche
- assemblage à crampon.

14. Agencement de bobines suivant l'une des revendications précédentes, dans lequel au moins un premier élément (303.1, 303.2, 303.3, 303.4, 303.5) d'antenne de la bobine (300) locale souple et au moins un deuxième élément (102.1, 102.2) d'antenne de la bobine (100) locale rigide sont découplés inductivement par chevauchement géométrique, lorsque la bobine (300) locale souple et la bobine (100) locale rigide constituent une bobine (500, 700) locale d'ensemble en forme de tube.
